# EUROPÄISCHE PATENTANMELDUNG

(11) **EP 2 447 242 A1**
(43) Veröffentlichungstag der Anmeldung: **02.05.2012**
(21) Anmeldenummer: 10189357.6
(22) Anmeldetag: 29.10.2010
(51) Int. Cl.: C07C 51/56, C07C 57/10, A23L 3/3508

(54) **Verfahren zur Herstellung von all-E-Sorbinsäureanhydrid**

(71) Anmelder: LANXESS Deutschland GmbH, 51369 Leverkusen (DE)
(72) Erfinder: Ritzer, Edwin, Dr., 51381 Leverkusen (DE); Vogl, Erasmus, Dr., 51373 Leverkusen (DE); Krahwinkel, Ralf, Dr., 40764 Langenfeld (DE); Markert, Robert, Dr., 51377 Leverkusen (DE)

(57) **Zusammenfassung**

Beschrieben wird ein Verfahren zur Herstellung von weitgehend isomerenreinem all-E-Sorbinsäureanhydrid, bei dem man Alkali-oder Erdalkalisalze der Sorbinsäure mit Phosgen in einem inerten organischen Lösungsmittel miteinander umsetzt. Weiterhin wird dessen Verwendung als Konservierungsmittel in Lebensmitteln, insbesondere Getränken beschrieben.

## Beschreibung

Gegenstand der Erfindung ist ein Verfahren zur Herstellung von weitgehend isomerenreinem all-E-Sorbinsäureanhydrid, das u.a. als Konservierungsmittel in Lebensmitteln Anwendung findet, sowie dessen Verwendung als Konservierungsmittel in Lebensmittel, insbesondere Getränken.

Es ist bekannt (z.B. aus der DE-A-102006035202 und WO 2008/014889), dass bestimmte Anhydride z.B. Benzoesäureanhydrid, aber auch Benzoesäure und Sorbinsäure selbst, ebenso wie deren Kaliumsalze auf Lebensmittel konservierende Eigenschaften haben. Kürzlich wurde in diesem Zusammenhang erkannt, dass das Sorbinsäureanhydrid eine etwa 5-fach stärker konservierende Wirkung als z.B. Kaliumsorbat aufweist. Diese Tatsache macht Sorbinsäureanhydrid als Sterilisator in z.B. Fruchtgetränken zu einem sehr interessanten Kandidaten.

Recherchiert man den Stand der Technik - im Hinblick auf Verfahren zur Herstellung von Sorbinsäureanhydrid - findet man Prozesse, die zunächst die Herstellung von Sorbinylchlorid beschreiben. Herstellwege von Sorbinylchlorid werden ausgehend vom Polyester der 3-Hydroxyhex-4-ensäure einem Vorprodukt der Sorbinsäureherstellung beschrieben (s. AT-A- 250928, GB-A-1077739, US -A-3322825, und DE-A- 1239681): 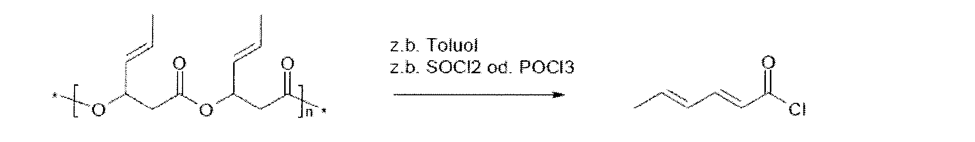 **Polyester der 3-Hydroxyhex-4-ensäure**
Molecular Weight =0.00[224.26]n
Molecular Formula =[C12H1604]n
CAS 26811-78-9

Eine phosgenbasierte Herstellung von Sorbinylchlorid findet sich in der DE-A-1931074:

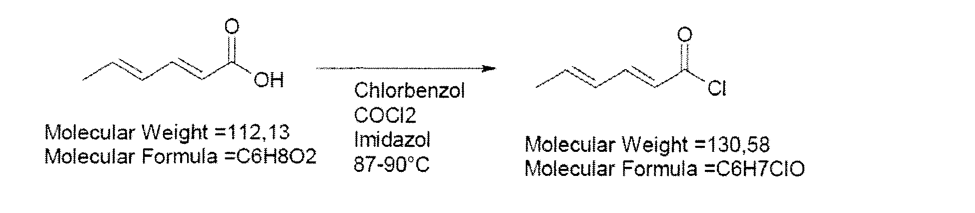

Nachteilig bei den oben beschriebenen Varianten ist vor allem die rasche Isomerisierung des Sorbinylchlorids unter den angegebenen Herstellbedingungen. Neben dem gewünschten all-E- Isomer findet man z.B. im Falle der Phosgenierung (DE-A-1931074 ) ungefähr 5-20 % weitere Stellungsisomere. Verursacht wird die Isomerisierung der all-E- Sorbinsäure vor allem durch die verfahrensgemäß freigesetzte Salzsäure und durch die Fahrweise bei erhöhter Temperatur.

Für die Polyesterspaltung liegen keine Erfahrungswerte zur Isomerisierung vor. Sie ist zudem stark an das spezielle Herstellverfahren des Polyesters gebunden und nicht überall praktikabel.

In beiden Fällen gelangt man also nicht zum all-E-Sorbinylchlorid, welches als Ausgangsstoff für all-E-Sorbinsäureanhydrid dienen könnte.

Zahlreiche weitere Herstellverfahren für Sorbinylchlorid liefert die allgemeine chemische Literatur; in allen Fällen sind dies Chlorierungen der Sorbinsäure mit Thionylchlorid, Oxalylchlorid etc. die die Isomerisierungsproblematik beinhalten.

Für Sorbinsäureanhydrid selbst lassen sich nur zwei Herstellwege recherchieren:

### 1) Herstellwege für Sorbinsäureanhydrid (s. hierzu DE-A-1283823, GB-A-1138813)

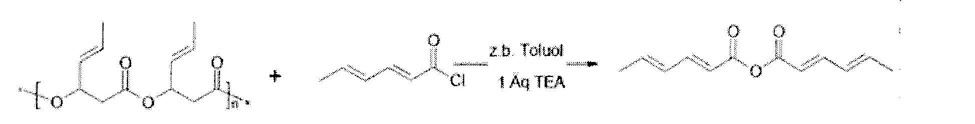

**Polyester der 3-Hydroxyhex-4-ensäure**
Molecular Weight =0.00[224.26]n
Molecular Formula=[C12H1604]n
CAS26811-78-9

Der Syntheseweg hat die schon weiter oben beschriebene Problematik des Polyesters und der Verfügbarkeit eines all-E-Sorbinylchlorids.

### 2) Eine auf Triphosgen zurückgehende Variante findet sich in CN-A-101357884 und lässt ebenfalls aufgrund der Reaktionsbedingungen (sauer, heiß) Isomerisierung erwarten.

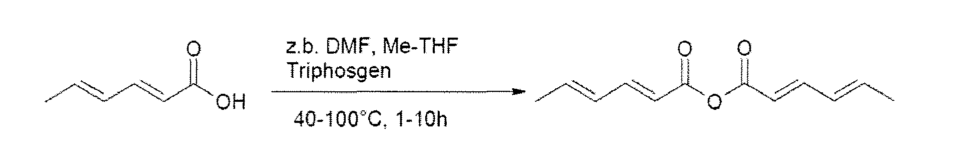

In der Literatur (Eckarkt, Cotarca, Phosgenations A Handbook, , Wiley-VCH 2003, p.353-357) findet man eine Variante zur Herstellung einiger, symmetrischer aliphatischer und aromatischer Anhydride, die auf der Phosgenierung von Carbonsäure-Triethylammoniumsalzen in inerten Lösungsmitteln basieren:

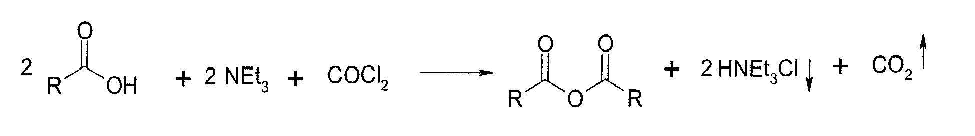

Die Anwendung dieser Prozedur auf das Triethylammoniumsalz der Sorbinsäure gelingt zwar prinzipiell (s. BSP1 (Vergleichsbeispiel)) hat aber wesentliche Nachteile. Hauptnachteil ist der große Anfall an Triethylammoniumchlorid. Dieses muss entweder abgetrennt (filtriert) und anschließend deponiert oder aber recycliert werden. Eine wässrige Abtrennung (Phasentrennung nach Verwässerung) ist zwar möglich, macht aber technisch die zusätzliche Entsorgung dieses Abwassers erforderlich. Auch führen im Produkt verbliebene geringfügige Aminspuren rasch zum Dunkeln und Verderben des erhaltenen Sorbinsäureanhydrids.

Nach dem derzeitigen Stand der Technik ist für die Herstellung von weitgehend isomerenreinem all-E-Sorbinsäureanhydrid demnach kein geeignetes Herstellverfahren zu finden.

Aufgabe der Erfindung war es, ein möglichst einfaches Verfahren zur Herstellung eines weitgehend isomerenreinen all-E-Sorbinsäureanhydrids zur Verfügung zu stellen.

Überraschenderweise wurde nun gefunden, dass die Phosgenierung von Alkali bzw. Erdalkalisalzen der Sorbinsäure (insbesondere des Kaliumsorbats) in inerten Lösemitteln bei geeigneten Temperaturen in einstufiger Synthese leicht und in hohen Ausbeuten gelingt:

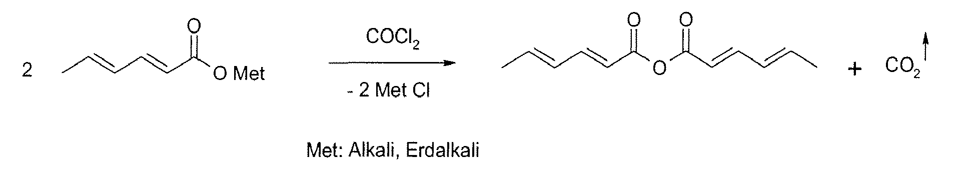

Gegenstand der Erfindung ist daher ein Verfahren zur Herstellung von weitgehend isomerenreinem Sorbinsäureanhydrid, bei dem man Alkali-oder Erdalkalisalze der Sorbinsäure mit Phosgen in einem inerten organischen Lösungsmittel miteinander umsetzt.

Im erfmdungsgemäßen Verfahren wird das gewünschte all-E- Sorbinsäureanhydrid in hoher Ausbeute und weitgehend isomerenrein erhalten. Unter "weitgehend isomerenrein" wird dabei verstanden, dass die anderen möglichen Isomeren nur in einer Menge im Bereich von 0,1-0,2 GC-Fl.% gebildet werden.

Im erfmdungsgemäßen Verfahren können alle Alkalisalze der Sorbinsäure wie das Lithium-,Natrium- oder Kaliumsalz und auch die Erdalkalisalze der Sorbinsäure wie das Barium-oder Calciumsalz eingesetzt werden. Bevorzugt sind das Natrium- und Kaliumsalz, besonders bevorzugt das Kaliumsalz der Sorbinsäure.

Das erfindungsgemäße Verfahren kann in jedem üblichen inerten organischen Lösungsmittel, wie es für Phosgenierungen eingesetzt wird durchgeführt werden. Beispiele für solche Lösungsmittel sind Toluol, Xylol, Chlorbenzol, Dichlorbenzol, Methyl-tert.butylether, Isomaylacetat, Butylacetat, Ethylacetat und Methylacetat. Bevorzugt setzt man Isoamylacetat, Butylacetat, Ethylacetat oder Methylacetat, besonders bevorzugt Ethylacetat oder Methylacetat als Lösungsmittel ein.

Die Reaktionstemperatur ist an sich unkritisch und sollte im Bereich zwischen -10 bis 50°C, bevorzugt im Bereich von -5 bis 20°C, besonders bevorzugt im Bereich von 0 bis 10°C liegen.

Mit der Ansatzstöchiometrie, d.h., dem Verhältnis von Sorbat: Phosgen kann die Bildung des weitgehend isomerenreinen all-E-Sorbinsäureanhydrids noch weiter optimiert werden. Üblicherweise sollte dieses Verhältnis im Bereich von 1:0,4 bis 1:0,6 liegen. Bevorzugt beträgt das Verhältnis 1:0,5, besonders bevorzugt 1.0,45.

Die Reaktionsdauer für die Phosgenierung liegt im Bereich von 2h bis 14 h.

Die Ausfällung des in molaren Mengen entstehenden Alkali- oder Erdalkalichlorids (z.B. Kaliumchlorid) ist beim erfindungsgemäßen Verfahren nicht störend und nicht gewässerkritisch. Mithin kann auch im technischen Maßstab die Salzfracht in einfacher Weise durch wässrige Phasentrennung entfernt werden.

Überraschend ist auch, dass das entstandene Anhydrid gegenüber der wässrigen Aufarbeitung nur wenig empfindlich ist, so dass im erfmdungsgemäßen Verfahren Ausbeuten von > 90 % d. Th. erhalten werden (vgl. die erfindungsgemäßen Beispiele 2 und 3).

Spuren von Alkali-oder Erdalkalichlorid wie z.B. Kaliumchlorid und Spuren von Sorbinsäure im Endprodukt stören die Anwendung als Konservierungsmittel nicht. Das erfindungsgemäße Verfahren zeichnet sich weiterhin dadurch aus, dass das nicht gewünschtes Sorbinylchlorid (ätzend und geruchsbelästigend!) nur im Bereich « 0,1 % anfällt. Es lässt sich durch die gewählte Ansatzstöchiometrie (Verhältnis Sorbat: Phosgen) fast vollständig vermeiden (vgl. erfindungsgemäßes Beispiel 3).

Ein weitere Gegenstand der Erfindung ist dabei die Verwendung von weitgehend isomerenreinem all-E-Sorbinsäureanhydrid als Konservierungsmittel für Lebensmittel, da sich gezeigt hat, das dieses Isomer eine ungefähr fünffach höhere Konservierungswirkung als Sorbinsäure oder Sorbat hat. Bevorzugt setzt man dafür nach dem erfmdungsgemäßen Verfahren hergestelltes all-E-Sorbinsäureanhxdrid ein. Bevorzugt werden Getränke erfindungsgemäß in den an sich üblichen Dosen für Konservierungsmittel konserviert.

Die Beispiele sollen die Erfindung weiter erläutern, ohne sie jedoch in ihrem Umfang zu begrenzen.

### Beispiele:

### Beispiel 1: (Vergleichsbeispiel) Phosgenierung von all-E-Sorbinsäure in Ethylacetat mit Triethylamin als Hilfsbase

In einem 1000 ml Vierhalskolben, ausgerüstet mit Thermometer, Intensivkühler, Tauchrohr für Phosgen und KPG-Rührwerk werden 100 g (0,88 mol) all-E- Sorbinsäure und 600 g Ethylacetat vorgelegt. Zu der erhaltenen Suspension werden 101,2 g (1 mol) Triethylamin gegeben worauf hin sich eine klare gelbe Lösung bildet. Der Ansatz wurde dann mit einem Kältebad auf 0-5°C abgekühlt. Anschließend wurden innerhalb von 4 Stunden bei 0-5°C 50,7 g (0,51 mol) Phosgen eingeleitet wobei sofort die Abscheidung des Triethylaminhydrochlorids beobachtet wird. Nach beendeter Phosgendosierung wurde der Ansatz über Nacht bei RT nachgerührt. Nach Prüfung auf Phosgenfreiheit wurde der Ansatz filtriert und der Nutschrückstand mit 200 ml Ethylacetat gewaschen. Das erhaltene, braungelbe Filtrat wurde am Rotationsverdampfer im Vakuum eingeengt. Das so erhaltene braune Öl (90,7 g) wurde unter Stickstoff in eine Klarglasflasche abgefüllt und erstarrte schnell bei RT. Eine GC-Analyse ergab einen Gehalt von 85,9 w% all-E-Sorbinsäureanhydrid; 9,13 w% unumgesetzte Sorbinsäure und 0,31 % Sorbinylchlorid. Im 1H-NMR wurde ein Gehalt von 94,5 w% Anhydrid und 2,9 w% Triethylamin festgestellt. Isomerisierung wurde nicht beobachtet. Bezogen auf den GC-Gehalt wurde eine Ausbeute von 85,6 % d.Th. bezogen auf eingesetzte Sorbinsäure ermittelt.

### Beispiel 2: (erfindungsgemäßes Beispiel) Phosgenierung von Kaliumsorbat in Ethylacetat (Stöchiometrie 1:0,5 eq. Phosgen)

In einem 6 ltr. Planschliffreaktor ausgerüstet mir Thermometer, Intensivkühler, Tauchrohr für Phosgen und KPG-Rührwerk wurden 600,87 g Kaliumsorbat (4 mol) und 2,4 kg Ethylacetat vorgelegt. Die erhaltene Suspension wird auf 0-10°C abgekühlt. Anschließend wurden 197,8 g Phosgen (2 mol) innerhalb von 4 Stunden eingeleitet. Nach dem Einleiten wurde der Ansatz ohne weitere Kühlung über Nacht nachgerührt. Nach Prüfung auf Phosgenfreiheit wurde der Ansatz (hellbeige Suspension) mit 1200 g VE-Wasser versetzt und gerührt. Nach Abstellen des Rührers wurde die untere, wässrige Phase abgetrennt und die obere, organische Phase am Rotationsverdampfer im Vakuum eingeengt. Es verbleiben 410,3 g eines gelben Öls, welches nach Abfüllen unter Stickstoff in eine Klarglasflasche rasch erstarrt. Die GC-Analyse des Materials ergab einen Gehalt an all-E-Sorbinsäureanhydrid von 95,8 w%, neben 0,13 % Isomer, sowie 3,3 w% Sorbinsäure und 0,64 % Sorbinylchlorid. Bezogen auf eingesetztes Kaliumsorbat entspricht dies einer Ausbeute von 95,3 % d.Th.

### Beispiel 3: (erfindungsgemäßes Beispiel) Phosgenierung von Kaliumsorbat in Ethylacetat (Stöchiometrie 1:0,45 eq. Phosgen)

In einem 6 ltr. Planschliffreaktor ausgerüstet mir Thermometer, Intensivkühler, Tauchrohr für Phosgen und KPG-Rührwerk wurden 2000 g Kaliumsorbat (13,31 mol) und 3,0 kg Ethylacetat vorgelegt. Die erhaltene Suspension wird auf 0-10°C abgekühlt. Anschließend wurden 595 g Phosgen (6,02 mol) innerhalb von 6 Stunden eingeleitet. Nach dem Einleiten wurde der Ansatz ohne weitere Kühlung über Nacht nachgerührt. Nach Prüfung auf Phosgenfreiheit wurde der Ansatz (hellbeige Suspension) beprobt. Ein GC des Reaktionsgemischs wies einen Gehalt von 28,9 w% all-E-Sorbinsäureanhydrid und 0,03 w% Sorbinylchlorid auf. Sorbinsäure und Isomeres Anhydrid waren nicht nachweisbar.

Zur Aufarbeitung wurde der Ansatz mit 3000 g VE-Wasser versetzt und gerührt. Nach Abstellen des Rührers wurde die untere, wässrige Phase abgetrennt und die obere, organische Phase am Rotationsverdampfer im Vakuum eingeengt. Es verbleiben 1154 g eines gelben Öls, welches nach Abfüllen unter Stickstoff in eine Klarglasflasche rasch erstarrt. Die GC-Analyse des Materials ergab einen Gehalt an all-E-Sorbinsäureanhydrid von 95 w%, neben 0,15 % Isomer, sowie 3,6 w% Sorbinsäure und nur 0,03 % Sorbinylchlorid. Bezogen auf eingesetztes Kaliumsorbat entspricht dies einer Ausbeute von 88,7 % d.Th.

## Patentansprüche

1. Verfahren zur Herstellung von weitgehend isomerenreinem all-E-Sorbinsäureanhydrid, bei dem man Alkali-oder Erdalkalisalze der Sorbinsäure mit Phosgen in einem inerten organischen Lösungsmittel miteinander umsetzt.

2. Verfahren nach Anspruch 1, **dadurch gekennzeichnet, dass** das Alkalisalz der Sorbinsäure Kaliumsorbat ist.

3. Verfahren nach Anspruch 1 oder 2, **dadurch gekennzeichnet, dass** das inerte organische Lösungsmittel ein Lösungsmittel aus der Gruppe Toluol, Xylol, Chlorbenzol, Dichlorbenzol, Methyl-tert-butylether, Isomaylacetat, Butylacetat, Ethylacetat und Methylacetat ist.

4. Verfahren nach einem oder mehreren der vorherigen Ansprüche, **dadurch gekennzeichnet, dass** die Reaktionstemperatur während der Phosgenierung im Bereich zwischen -10 bis 50°C, bevorzugt im Bereich zwischen -5 bis 20°C, besonders bevorzugt im Bereich zwischen 0 bis 10°C liegt.

5. Verfahren nach einem oder mehreren der vorherigen Ansprüche, **dadurch gekennzeichnet, dass** das Verhältnis von Sorbat : Phosgen im Bereich zwischen 1:0,6 bis 1:0,4eq.liegt , bevorzugt 1:0,5 eq., besonders bevorzugt 1:0,45 eq. beträgt.

6. Verfahren nach einem oder mehreren der vorherigen Ansprüche, **dadurch gekennzeichnet, dass** der Anteil der entstehenden nicht all-E-Sorbinsäureanhydrid-Isomeren im Bereich von 0,1 bis 0,2GC-F1. % liegt.

7. Verwendung von weitgehend isomerenreinem all-E-Sorbisäureanhydrid als Konservierungsmittel für Lebensmittel.

8. Verwendung nach Anspruch 7, **dadurch gekennzeichnet, dass** das all-E-Sorbinsäureanhydrid nach einem oder mehreren der vorherigen Ansprüche 1 bis 6 erhalten wurde.
